# EUROPEAN PATENT SPECIFICATION

(11) **EP 2 467 471 B1**
(45) Date of publication and mention of the grant of the patent: **13.01.2016**
(21) Application number: 10737274.0
(22) Date of filing: 16.07.2010
(51) Int. Cl.: C12N 7/02, A23L 3/358

(54) **METHOD FOR ISOLATING VIRUSES**
VERFAHREN ZUR VIRUSISOLATON
MÉTHODE D'ISOLEMENT DE VIRUS

(30) Priority: 17.08.2009 EP 09010584
(43) Date of publication of application: 27.06.2012
(73) Proprietor: Merck Patent GmbH, 64293 Darmstadt (DE)
(72) Inventor: ROSSMANITH, Peter, A-2531 Gaaden (AT); MESTER, Patrick, Julian, 1020 Wien (AT); HUEHN, Stephan, 14163 Berlin (DE); WAGNER, Martin, A-1180 Wien (AT)
(86) International application number: PCT/EP2010/004334
(87) International publication number: WO 2011/020530

(56) References cited:
- HERRMANN ET AL: "FOOD-BORNE VIRUS: DETECTION IN A MODEL SYSTEM", APPLIED MICROBIOLOGY, vol. 16, no. 4, 1968, pages 595-602, XP002614764,
- KIM H Y ET AL: "Optimization of methods for detecting norovirus on various fruit", JOURNAL OF VIROLOGICAL METHODS, vol. 153, no. 2, 1 November 2008 (2008-11-01), pages 104-110, XP025495474, ELSEVIER BV, NL ISSN: 0166-0934, DOI: 10.1016/J.JVIROMET.2008.07.022 [retrieved on 2008-09-10]
- CROCI ET AL: "CURRENT METHODS FOR EXTRACTION AND CONCENTRATION OF ENTERIC VIRUSES FROM FRESH FRUIT AND VEGETABLES: TOWARDS INTERNATIONAL STANDARDS", FOOD ANAL. METHODS, vol. 1, 2008, pages 73-84, XP002614765,
- EARLE M J ET AL: "Ionic liquids. Green solvents for the future", PURE & APPLIED CHEMISTRY, vol. 72, no. 7, 1 January 2000 (2000-01-01), pages 1391-1398, XP002266043, PERGAMON PRESS, OXFORD, GB ISSN: 0033-4545
- Anonymous: "ILCO Ionic Lube", ILCO Chemikalien, 2008, XP002628011, Retrieved from the Internet: URL:http://www.ilco-chemie.de/downloads/Io nic%20Liquid.pdf [retrieved on 2011-03-10]

## Description

The present invention relates to a method for the isolation of viruses from a sample. The sample is treated with an extraction solution that comprises at least a divalent chloride salt optionally in combination with an ionic liquid resulting in the isolation of the viruses.

### Background of the invention

The isolation of viruses from complex samples for their identification or characterisation or simply for further processing is becoming increasingly important, in particular the identification of pathogens in samples like food samples or clinical samples like blood, tissue or feces. However, in order to clearly identify and optionally to quantify the viruses comprised in a sample methods for their isolation have to be provided.

In contrast to other microorganisms like bacterial cells, which can be multiplied prior to their detection, most virus particles need to be directly detected in the amount present in the respective sample. For this reason it is even more important to generate very sensitive methods which enable the detection and identification of only very few copies of one single virus in a sample.

Real-time PCR has greatly enhanced the application field of PCR as a quantitative tool in molecular biology in general and for the quantification and identification of microorganisms or viruses, in particular of pathogens. Real-time PCR allows the reliable detection and quantification down to one single nucleic acid target per PCR sample but requires highly purified template nucleic acids. Especially when it comes to routine diagnostics and quantitative detection of cells or viruses in complex environments like food these requirements play a key role as inhibitory effects caused by components of these environments may influence or even inhibit the PCR reaction. Furthermore it is crucial to use a reliable and efficient recovery method to be used for the isolation of the target organisms from complex samples like food. Since samples like food involve generally large sample volumes microbiological methods are normally used for microorganism isolation and enrichment. These methods represent the "golden standard" methods and new alternative techniques have to be evaluated in comparison to them.

Major efforts have been made to establish methods for the separation of microorganisms, e.g. of bacteria, from food which meet the demanding requirements of real time PCR and other molecular methods for downstream analysis of the microorganisms.

Also the isolation of nucleic acids directly out of food has been attempted using nucleic acid isolation methods commonly used in molecular biology. Other methods utilize the affinity of biomolecules to surface structures of microorganisms, whereby said biomolecules may be, for instance, antibodies, bacteria binding proteins from phages and antimicrobial peptides (AMPs) optionally in combination with magnetic beads, silanized glass slides or direct colony blot. For instance, for the direct detection of Listeria monocytogenes an aqueous two-phase separation system can be used (Lantz et al. Appl Environ Microbiol. (1994) 60:3416-3418).

Most of these methods have drawbacks like insufficient size of processed sample volume, high detection limits, low recovery rates, no quantitative isolation of cells, time consuming procedure and high costs. In addition the application of these methods has been restricted in most cases to only one or a limited number of different food matrices. Based on the requirements for direct quantification of pathogens in food which are (i) a large sample volume, (ii) a reproducible recovery rate over a broad range of target concentrations, and (iii) removal of inhibitors to aid alternative molecular methods for downstream analysis, new protocols for isolation of pathogens like viruses or bacteria have to be provided.

WO 2008/017097 discloses a method for isolating cells being surrounded by a cell wall from complex matrices like foodstuff. This method uses an extraction buffer comprising a chaotropic agent in combination with a detergent.

As can be seen from the above mentioned known methods, most methods are directed to the isolation of larger microorganisms like bacterial cells. While bacterial cells are quite large objects which can be followed up and recovered from a sample quite easily, none of the known procedures offers an easy and effective way for the isolation of small objects like viruses.

The prior art also includes Herrmann et al, Applied Microbiology, 16, 1968, 595-602. This discloses the extraction of a 'model' contaminating virus (a cocksackie virus) from cottage cheese. Sample was homogenized after preparation in a buffer containing, inter alia, MgCl2 at about 1 M (table 2). Following homogenization, various concentration and centrifugation steps were performed, and virus tested in tissue culture. Homogenization was performed for 2 minutes.

In L. Croci et al., Food Anal. Methods (2008), 1, 73-84, the authors discuss various attempts to extract and concentrate viruses from food samples. They come to the conclusion that viral contamination is increasingly identified as cause of food borne illnesses, like illnesses caused by norovirus, hepatitis A virus, rotavirus or enterovirus. Nevertheless, it is concluded that - though several attempts have been made to extract viruses - more sensitive, reliable and standardized methods are needed.

Consequently, there exists a clear need for quantitative and reproducible methods for the isolation of viruses from complex matrices like food and clinical samples.

### Brief description of the Invention

It has been found that viruses can easily and very effectively be isolated from complex matrices using a buffer which comprises at least a divalent chloride salt optionally in combination with an ionic liquid. Surprisingly, the addition of the above mentioned substance or substances results in such an effective lysis of the complex sample matrix that even viruses which typically tend to "stick" to the sample matrix can be isolated.

The present invention relates to a method for isolating viruses as set out in the claims.

The disclosure also relates to a kit for the isolation of viruses from a complex sample comprising
- an extraction solution comprising at least a divalent chloride salt and/or an ionic liquid
   and
- at least one biodegrading enzyme

### Description of the invention

It surprisingly turned out that the incubation of a complex sample with an extraction solution that comprises at least a divalent chloride salt optionally in combination with an ionic liquid results in such an effective dissolution of the sample that even viral particles are set free without being destroyed.

The method according to the present invention may be used to isolate viruses, also called virus particles. Viruses are known to a person skilled in the art. A complete virus particle consists of nucleic acid surrounded by a protective coat of protein called a capsid. These are typically formed from identical protein subunits called capsomers. The shape of the capsid can serve as the basis for the morphological distinction of the viruses. In general, there are four main morphological virus types:
- Helical

Tobacco mosaic virus is an example of a helical virus.
- Icosahedral
- Envelope

The influenza virus and HIV use this strategy.
- Complex

A person skilled in the art knows the different types of viruses.

The present invention allows isolation viruses in general, preferably food and pathogen viruses, especially those of relevance for humans, e.g. those potentially present in human food or pathogens with clinical relevance. Some exemplary viruses which are of special interest are listed below. They are either of epidemiological importance like Influenza (Orthomyxoviridae), HIV 1 + 2 (Orthoretroviridae), Pox (Poxviridae, Orthopoxviridae), Corona (Coronaviridae, Nidovirales), Flavivirus, Polyomavirus or Papiloma virus or they can be found as contaminants especially in food samples like Adenoviruses (Adenoviridae), Rotaviruses (Rheoviridae), Enteroviruses, Noroviruses, Norwalk / Norwalk-like viruses (Caliciviridae), Hepatitis A viruses (Picornaviridae), Hepatitis E viruses (Hepeviridae) or Astroviruses.

The size (= diameter) of the viral particles to be isolated according to the present invention is typically between 10 and 500 nm, preferably between 10 and 200 nm, more preferably between 10 and 100 nm.

The term "complex sample" refers to a sample or sample matrix comprising a greater or lesser number of different compounds of mainly organic origin, which may be liquid and/or solid. A complex sample according to the present invention typically comprises a matrix comprising peptides, polypeptides, proteins (including also enzymes), carbohydrates (complex and simple carbohydrates), lipids, fatty acids, fat, nucleic acids etc.. A "complex sample" can also comprise one or more substances which interfere with the isolation and/or detection of the viruses, e.g. by inhibiting amplification of the viral nucleic acids. In the invention, the "complex sample" is a food or clinical sample.

Complex samples include, but are not limited to, food (e.g. milk of cows, ewes, nanny goats, mares, donkeys, camels, yak, water buffalo and reindeer, milk products, meat of beef, goat, lamb, mutton, pork, frog legs, veal, rodents, horse, kangaroo, poultry, including chicken, turkey, duck, goose, pigeon or dove, ostrich, emu, seafood, including finfish such as salmon and tilapia, and shellfish such as mollusks and crusta ceans and snails, meat products, plant products, seeds, cereals from grasses, including maize, wheat, rice, barley, sorghum, and millet, cereals from non-grasses, including buckwheat, amaranth, and quinoa, legumes, including beans, peanuts, peas, and lentils, nuts, including almonds, walnuts, and pine nuts, oilseeds, including sunflower, rape and sesame, vegetables like root vegetables, including potatoes, cassava and turnips, leaf vegetables, including amaranth, spinach and kale, sea vegetables, including dulse, kombu, and dabberlocks, stem vegetables, including bamboo shoots, nopales, and asparagus, inflorescence vegetables, including globe artichokes, broccoli, and daylilies, and fruit vegetables, including pumpkin, okra and eggplant, fruits, herbs and spices, whole blood, urine, sputum, vomit, saliva, amniotic fluid, plasma, serum, pulmonary lavage and tissues, including but not limited to, liver, spleen, kidney, lung, intestine, brain, heart, muscle, pancreas and the like. The skilled artisan will appreciate that lysates, extracts or (homogenized) material obtained from any of the above exemplary samples or mixtures of said exemplary samples or compositions comprising one or more of said exemplary samples are also samples within the scope of the disclosure made.

According to the present invention, the term "divalent chloride salt" means chlorides of divalent cations, like MgCl₂, CaCl₂, SrCl₂, ZnCl₂ or MnCl₂. Most preferred divalent chloride salts are MgCl₂ and ZnCl₂.

The term "buffer" or "buffer solution" as used herein, refers to aqueous solutions or compositions that resist changes in pH when acids or bases are added to the solution or composition. This resistance to pH change is due to the buffering properties of such solutions. Thus, solutions or compositions exhibiting buffering activity are referred to as buffers or buffer solutions. Buffers generally do not have an unlimited ability to maintain the pH of a solution or composition. Rather, they are typically able to maintain the pH within certain ranges, for example between pH 7 and pH 9. Typically, buffers are able to maintain the pH within one log above and below their pKa (see, e.g. C . Mohan, Buffers, A guide for the preparation and use of buffers in biological systems, CALBIOCHEM, 1999). Buffers and buffer solutions are typically made from buffer salts or preferably from non-ionic buffer components like TRIS and HEPES. The buffer added to the extraction solution guarantees that the pH value in the course of the matrix dissolution will be stabilized. A stabilized pH value contributes to reproducible results, efficient lysis and conservation of the isolated cells.

As used herein, the term "detergent" refers to molecules having lipophilic as well as hydrophilic (i.e. amphiphilic) characteristics. A detergent according to the present invention may comprise, for instance, a fatty acid residue and a hydrophilic (e.g. anionic or cationic) part.

According to the disclosure made the sample may be a food sample, feces, a body fluid, in particular blood, plasma or serum, water or a tissue sample.

Particularly preferred samples are samples with a complex matrix (i.e. comprising among others proteins, lipids, carbohydrates etc.) and/or a high viscosity.

The food sample is preferably a milk product, preferably milk, in particular raw milk, milk powder, yoghurt, cheese or ice cream, a fish product, preferably raw fish, a meat product, preferably raw meat, meat rinse or sausages, salad rinse, chocolate, egg or egg products, like mayonnaise, salad, sea food, preferably mussels, fruits, preferably berries, and peppers. Particularly preferred food samples used in the method according to the present invention are samples which are usually known to comprise potentially pathogenic viruses and from which viruses are - due to a complex matrix - hardly extractable or detectable with the methods known in the art. In particular cheese is known as a food with a complex matrix and high viscosity.
Particularly preferred clinical samples are feces and blood. Samples also include cell culture samples.

According to the present invention, the extraction solution used as matrix lysis system comprises a divalent chloride salt optionally in combination with an ionic liquid. It has been found that depending on the type of the sample and depending on the type of the virus, different compositions of the extraction solution are most suitable.
For example, dairy products with a complex matrix that can be dissolved or extracted under milder conditions are preferably treated with an extraction solution comprising MgCl₂.

Samples which comprise higher amount of starch (more than 5% w/w) or meat samples are preferably extracted or solubilised with an extraction solution comprising ZnCl₂ in concentrations between 5 and 10 M.

The divalent chloride salts like MgCl₂ are typically present in concentrations between 0.5 and 6 M, preferably between 0.5 and 4 M, more preferably between 1 and 2 M.

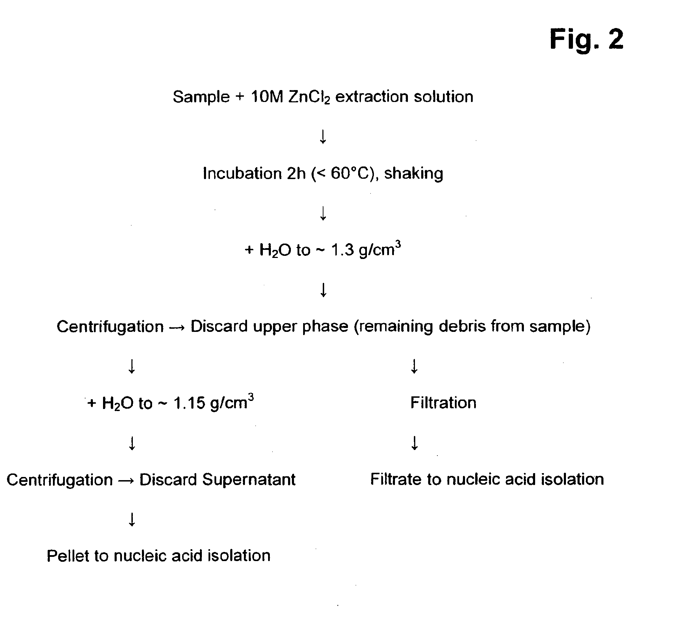

ZnCl₂ can also be used in higher concentrations due to its very high water solubility. It can be used in concentrations up to about 15 M. Preferred ZnCl₂ concentrations are between 1 and 10 M. This offers the possibility to optionally in combination with an ionic liquid. create very specific extraction conditions and to directly use the extraction solution for gradient centrifugation. An example of an extraction protocol with ZnCl₂ is shown in Figure 2. After incubation of the sample with an extraction solution comprising ZnCl₂, the density of the mixture is adjusted to a magnitude suitable for gradient centrifugation by the addition of water. After this first centrifugation step to remove sample debris and other impurities, the remaining sample can be diluted again to adjust the density for another centrifugation step or it can alternatively be subjected to a filtration step.

The ionic liquid - if present - is typically present in concentrations between 0.5 and 100% by weight, preferably between 1 and 60% by weight, more preferably between 7 and 40 % by weight, based on the weight of mixture. The ionic liquid can be one ionic liquid or a mixture of two or more ionic liquids.

The best concentration of the divalent chloride salt optionally in combination with an ionic liquid mainly depends on the sample to be dissolved and the viral species to be isolated. These parameters can be tested easily by the person skilled in the art.

The extraction solution for use in the present invention is typically an aqueous solution and/or a buffer solution which may comprise one or more organic solvents, preferably one or more water-miscible solvents like ethanol or methanol, also comprising at least a divalent chloride salt and/or an ionic liquid. It typically has a pH value greater than 5 and lower than 11, preferably greater than 6 and lower than 9, more preferably between 6.5 and 7.5. Preferably the extraction solution comprises water, a buffer solution or a mixture of water or a buffer solution with up to 50% (v/v) of one or more water-miscible organic solvents and at least a divalent chloride salt in a concentration between 0.5 and 6m.

The buffer which may be used in the method of the present invention is preferably selected from the group of phosphate buffer, phosphate buffered saline buffer (PBS), 2-amino-2-hydroxymethyl-1, 3-propanediol (TRIS) buffer, TRIS buffered saline buffer (TBS), TRIS/EDTA (TE), ACES, MES, PIPES, HEPES and Tricine. Preferred buffers are PBS and Tricine.

In order to achieve an even better dissolution of the sample, said sample may additionally be incubated with at least one detergent, preferably an anionic detergent and/or a zwitterionic detergent and/or a nonionic detergent. The detergent can be added to the sample to reach a final concentration in the mixture of 0.01% to 5%, preferably 0.1% to 3%, more preferably 0.2% to 2% (% by weight).

The anionic detergent is preferably sodium dodecyl sulfate (SDS), lithium dodecyl sulfate (LDS) or deoxycholate (DOC).

The zwitterionic detergent is preferably 3- [(3-cholamidopropyl) dimethylammonio] -1-propanesulfonate (CHAPS) or 3- [(3-Cholamidopropyl)dimethylammonio]-2-hydroxyl-propanesulfonate (CHAPSO).

The nonionic detergent is preferably an ethoxylated aliphatic alcohol, preferably comprising a C13 to C15 aliphatic alcohol. Such ethoxylated aliphatic alcohols are also known as Lutensol. Suitable nonionic detergents are, in particular, acyl-, alkyl-, oleyl- and alkylarylethoxylates . These products are obtainable, for example on the market under the name Genapol or Lutensol. This covers, for example, ethoxylated mono-, di- and trialkylphenols (EO (ethyleneoxy group) degree: 3 to 50, alkyl substituent radical: C 4 to C12) and also ethoxylated fatty alcohols (EO degree: 3 to 80; alkyl radical: C8 to C36), espe-cially C12-C14-fatty alcohol (3-8) ethoxylates, Cl3-C15-oxo alcohol (3-30) ethoxylates, C16-C18-fatty alcohol (11-80) eth-oxylates, ClO-oxo alcohol (3-11) ethoxylates, C13-oxo alcohol (3-20) ethoxylates, polyoxyethylenesorbitan monooleate having 20 ethylene oxide groups, copolymers of ethylene oxide and propylene oxide having a minimum content of 10% by weight of ethylene oxide, the polyethylene oxide (4-20) ethers of oleyl alcohol and also the polyethene oxide (4-20) ethers of nonyl phenol. Use may also be made of mixtures of said nonionic detergents.

It is of course possible to add to the extraction solution one or more additional substances like destabilizing agents or biopolymer degrading enzymes which help to degrade substances present in specific samples. As discussed below, one example is the addition of starch degrading enzymes for food samples comprising high amounts of collagen and/or starch.

The incubation is typically performed at temperatures between 18°C and 98°C, preferably between 25°C and 80°C, more preferably between 35°C and 70°C.

The sample is typically incubated with the extraction solution for a time between 10 minutes and 6 hours, preferably between 20 minutes and 1 hour.

In order to dissolve the sample even more efficiently and in a reduced time, it is advantageous to perform the incubation at an elevated temperature.

It surprisingly turned out that the matrix lysis according to the present invention is efficient enough to allow the isolation of viruses after the lysis. The viruses can be isolated by any known method. Preferred methods are centrifugation, filtration, sieving, gel electrophoresis, gel filtration, dielectrophoresis, precipitation like precipitation with polyethylenglycol or immunprecipitation, solvent extraction (with 2 or 3 phases) and ultrasound or affinity binding, e.g. using antibodies, lectins, proteins binding viruses or aptamers which are preferably immobilized e.g. on beads.

The methods can also be combined. Preferably, the viruses are primarily isolated by filtration, sieving or centrifugation, most preferably by centrifugation. If necessary, after the centrifugation step, the viruses can be finally isolated by precipitation.
In one embodiment, this is done by performing a first isolation step like centrifugation, then coagulating the viruses to form clusters, e.g. by adding suitable antibodies, and then isolating the viruses by precipitation.

Centrifugation is typically carried out at 500 to 300.000 g , more preferably at more than 1.000 g , even more preferably at more than 20.000 g.

In a preferred embodiment, a stepwise centrifugation procedure is performed. In a first centrifugation step with a centrifugation typically between 100 and 3.500 g, most of the sample matrix and additional components like bacterial cells are removed. The remaining supernatant comprising at least the viral particles can be additionally centrifuged to isolate the viral particles. This is typically performed with a centrifugation at 10.000 to 300.000 g depending on the size and the type of the virus.

Alternatively, after removal of the sample matrix and additional components (e.g. by centrifugation), the remaining supernatant can be treated with components suitable to precipitate viruses. Examples of such components are Al₂(SO₄)₃, Coomassie Brilliant Blau (R oder G), ZnCl₂, MgCl₂, NaPO₄, ZnSO₄, Ammoniumchloride or polyethylene glycole. The clustered viruses can then be isolated by centrifugation typically between 1.000 and 20.000 g. It has been found that for the method according to the present invention MgCl₂ and especially ZnCl₂ are preferred components for the precipitation of viruses. A person skilled in the art can easily determine the amount of the component necessary to precipitate the viruses. ZnCl₂ and MgCl₂ are typically applied in an amount resulting in concentrations of more than 3 mol/l, preferably around 4 to 5 mol/l.

If bacterial cells and viral particles shall be isolated in parallel, the sample can be centrifuged at low speed (typically between 100 and 500 g) to remove the sample matrix. The supernatant can then be treated with components suitable to precipitate viruses. The clustered viruses and the bacterial cells can then be isolated by centrifugation typically between 1.000 and 20.000 g.

The stepwise centrifugation method is one preferred way to use the method of the invention to not only isolate viruses but isolate bacterial cells or other cells surrounded by a cell wall and viruses from one sample.
Cells surrounded by a cell wall and viruses have totally different properties but it has been found that the method according to the present invention for the first time offers the possibility to isolate both species in parallel - but with the possibility to isolate them one after the other or simultaneously together.

The extraction solution for use according to the present invention are in principle, also suitable for the isolation of cells surrounded by a cell wall like preferably bacterial cells. The extraction solutions comprising divalent chloride salts, preferably MgCl₂ optionally in combination with an ionic liquid even offer the possibility to isolate viable bacterial cells.

The term "cells surrounded by a cell wall" refers to all cells known having or comprising a cell wall as a barrier to the environment. Examples for organisms or cells having a cell wall are bacteria, archaea, fungi, plants and algae. In contrast thereto, animals and most other protists have cell membranes without surrounding cell walls.
As used herein, "viable cells" include cells with active metabolism, preferably propagable, especially cells which are able to multiply.

The bacterial cells to be isolated with the method according to the present disclosure are e.g. gram-negative or gram positive cells, most preferably selected from the group consisting of Listeria spp., S. aureus, P. paratuberculosis, Salmonella spp. or C . jejuni.

If the sample/extraction solution mixture is filtered or sieved the viruses are retained on the surface of said filter, sieve or gel, when the pore size of the filter is adapted to the size of the viral particles to be isolated. Of course it is also possible to apply more than one filtration step with different filters having varying pore sizes. After the filtration step the viruses can be washed from the filter surface (see e.g. Stevens KA and Jaykus L-A, Crit Rev Microbiol (2004) 30:7-24). Filtration of the lysed sample is in particular required when the complex sample comprises material which will hardly or not be lysed with the method of the present invention.
Typically these materials comprise starch and/or fibers.

However, the preferred method for isolating the viruses from the lysis mixture is centrifugation or centrifugation combined with precipitation.

Of course it is also possible to isolate the viruses from the dissolved pellet formed after the centrifugation step by immunological methods involving antibodies, in particular antibodies immobilized on beads, preferably magnetic beads, which are directed to epitopes present on the viruses to be isolated. Since the use of antibody beads for isolating viruses results in some cases in a reduced recovery rate, such methods may preferably employed mainly for qualitative isolation.

In order to facilitate the dissolution of the sample, said sample can be, for instance, homogenized using a stomacher prior to its incubation with the extraction solution. The dissolution is further supported and/or accelerated when the sample/extraction solution mixture is agitated during the incubation.

The incubation step may - depending on the sample matrix - be repeated once or several times, e.g. twice, three times, four times, five times or ten times. Between these incubation steps the viruses and the remnant sample matrix may be separated from the supernatant by e.g. centrifugation.

The viruses isolated with the method according to the present invention may be used for quantitatively and/or qualitatively determining the viruses in the sample. This can be achieved, for instance, by cell counting, by PCR methods, in particular by real time PCR, by using lectins or by methods involving antibodies, proteins selectively binding viruses or aptamers directed to surface structures of said virus particles (e.g. particle specific ELISA or RIA).
After the isolation step the viruses are preferably washed with water, a buffer solution and/or detergent comprising solutions. However, it is of course possible to add to the wash buffer one or more additional substances. The wash step may be repeated for several times (e.g. 2, 3 , 4, 5 or 10 times) or only once. In the course of the washing step the viruses are typically resuspended in the buffer and then filtered or centrifuged. If insoluble particles are present in the dissolved sample (e.g. calcium phosphate particles of cheese) said particles can be removed either by centrifugation at a lower rotational speed or by letting the particles settle over time (viruses will remain in both cases in the supernatant).

The viruses may also be washed with detergent comprising solutions. This will allow to further remove fat remnants potentially contained in the cell suspension. Preferred detergents to be used in this method step are those detergents regularly used for fat removal.

According to a preferred aspect of the present disclosure the amount of the viruses in the sample is determined.

The amount of the viruses in the sample can be determined by any method known in the art, in particular by methods like dilution series, phage count, real time PCR/real time RT PCR etc..

According to another preferred embodiment of the present invention the DNA or RNA of the viruses is isolated.
Depending on the viruses various methods may be employed to extract DNA (e.g. genomic DNA, plasmids) or RNA (e.g. mRNA). All these methods are known in the art and the single protocols mainly depend on the viruses to be lysed.

In order to determine or to monitor the efficiency of the isolation procedure the sample can be spiked with a defined amount of control viruses. The control viruses are typically inactivated viral particles. Preferably they are similar to the viruses assumed to be present in the sample but they are preferably not identical to the viruses assumed to be present in the sample. The amount of the recovered spiked control viruses allows to determine the efficiency of the method of the present invention and may also indicate the amount of the viruses to be isolated and determined present in the initial sample.

According to one aspect of the present disclosure the sample is further incubated with at least one biopolymer degrading enzyme.

Some samples from which the viruses are isolated comprise structures of biopolymers which may not or only in an inefficient manner be lysed by the addition of the extraction solution. If the sample, in particular the food sample, for example comprises collagen and/or starch in an amount of, e.g., over 10%, said sample may be treated with substances capable of degrading at least partially the collagen and starch content prior to its incubation with the matrix lysis system of the present disclosure.

Therefore the sample is preferably incubated further with at least one biopolymer degrading enzyme. Samples which are preferably incubated with biopolymer degrading enzymes are e.g. meat, fish, etc. Ice cream, eggs, blood, milk, milk products etc. do usually not require the addition of biopolymer degrading enzyme. It surprisingly turned out that the use of enzymes alone does not allow the isolation of viruses.

As used herein, the term "biopolymer" refers to proteins, polypeptides, nucleic acids, polysaccharides like cellulose, starch and glycogen etc. Therefore a "biopolymer degrading enzyme" is an enzyme which is able to degrade a biopolymer (e.g. starch, cellulose), which may be insoluble in an aqueous buffer, to low molecular substances or even to monomers.
Since the biopolymer degrading enzyme may be active under certain pH and temperature conditions (the use of specific buffers may also play a role) it is advantageous to perform the incubation with said enzymes under optional conditions. These conditions depend on the enzyme used and are known in the art. Also the incubation time depends on extrinsic factors like pH and temperature. Therefore the incubation time may vary from 10s to 6h, preferably 30s to 2h.

The biopolymer degrading enzyme is preferably selected from the group consisting of proteases, cellulases and amylase. Examples of these enzymes are Savinase 24 GTT (Subtilin), Carenzyme 900 T , Stainzyme GT. Starch degrading enzymes are e.g. cyclodextrin glucanotransferase, alpha-amylase, beta-amylase, glucoamylase, pullulanase and isoamylase, in particular α-amylase.

In known methods using buffers comprising chaotropic agents and detergents the biopolymer degrading enzymes cannot be added during the matrix lysis step as chaotropes and detergents may negatively influence the enzyme activity so that the biopolymers are not efficiently degraded into fragments or monomers.

In contrast to this, in the method according to the present invention where divalent chloride salts optionally in combination with an ionic liquid are used in the extraction solution, the biopolymer degrading enzyme can be incubated with the sample prior to step b) and/or during step b) and/or after step c) (step b) being the lysis step where the sample is incubated with the extraction solution and step c) being the isolation step).

The method according to the present invention can be performed within a few hours, typically within 1 to 6 hours.

Ionic liquids or liquid salts as used in the present invention are ionic species which consist of an organic cation and a generally inorganic anion. They do not contain any neutral molecules and usually have melting points below 373 K.

The area of ionic liquids is currently being researched intensively since the potential applications are multifarious. Review articles on ionic liquids are, for example, R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse" [Ionic Liquids - Novel Solutions for Transition-Metal Catalysis], Angew. Chem., 112 (2000), 3926-3945; T. Welton "Room temperature ionic liquids. Solvents for synthesis and catalysis", Chem. Rev., 92 (1999), 2071-2083 or R. Hagiwara, Ya. Ito "Room temperature ionic liquids of alkylimidazolium cations and fluoroanions", J. Fluorine Chem., 105 (2000), 221-227).

In general, all ionic liquids of the general formula K⁺A⁻ known to the person skilled in the art, in particular those which are miscible with water, are suitable in the method according to the invention.

The anion A- of the ionic liquid is preferably selected from the group comprising halides, tetrafluoroborate, hexafluorophosphate, cyanamide, thiocyanate or imides of the general formula [N(R_{f})₂]⁻ or of the general formula [N(XR_{f})₂]⁻, where R_{f} denotes partially or fully fluorine-substituted alkyl having 1 to 8 C atoms and X denotes SO₂ or CO. The halide anions here can be selected from chloride, bromide and iodide anions, preferably from chloride and bromide anions. The anions A- of the ionic liquid are preferably halide anions, in particular bromide or iodide anions, or tetrafluoroborate or cyanamide or thiocyanate, most preferred thiocyanate.

There are no restrictions per se with respect to the choice of the cation K⁺ of the ionic liquid. However, preference is given to organic cations, particularly preferably ammonium, phosphonium, uronium, thiouronium, guanidinium cations or heterocyclic cations.

Ammonium cations can be described, for example, by the formula (1)

[NR₄]⁺ (1),

where
R in each case, independently of one another, denotes
H, where all substituents R cannot simultaneously be H,
OR', NR'₂, with the proviso that a maximum of one substituent R in formula (1) is OR', NR'₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more R may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' may be = H, non-, partially or perfluorinated C₁- to C₆-alkyl, C₃- to C₇-cycloalkyl, unsubstituted or substituted phenyl and X may be = halogen.

Phosphonium cations can be described, for example, by the formula (2)

[PR²₄]⁺ (2),

where
R² in each case, independently of one another, denotes H, OR' or NR'₂
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more R² may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R² which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

However, cations of the formulae (1) and (2) in which all four or three substituents R and R² are fully substituted by halogens are excluded, for example the tris(trifluoromethyl)methylammonium cation, the tetra(trifluoromethyl)ammonium cation or the tetra(nonafluorobutyl)ammonium cation.

Uronium cations can be described, for example, by the formula (3)

[(R³R⁴N)-C(=OR⁵)(NR⁶R⁷)]⁺ (3),

and thiouronium cations by the formula (4),

[(R³R⁴N)-C(=SR⁵)(NR⁶R⁷)]⁺ (4),

where
R³ to R⁷ each, independently of one another, denotes
hydrogen, where hydrogen is excluded for R⁵,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R³ to R⁷ may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R³ to R⁷ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-where R' = H, non-, partially or perfluorinated C₁- to C₆-alkyl, C₃- to C₇-cycloalkyl, unsubstituted or substituted phenyl and X = halogen.

Guanidinium cations can be described by the formula (5)

[C(NR⁸R⁹)(NR¹⁰R¹¹)(NR¹²R¹³)]⁺ (5),

where
R⁸ to R¹³ each, independently of one another, denotes
hydrogen, -CN, NR'₂, -OR'
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R⁸ to R¹³ may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R⁸ to R¹³ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-where R' = H, non-, partially or perfluorinated C₁- to C₆-alkyl, C₃- to C₇-cycloalkyl, unsubstituted or substituted phenyl and X = halogen.

In addition, it is possible to employ cations of the general formula (6)

[HetN]⁺ (6),

where
HetN⁺ denotes a heterocyclic cation selected from the group 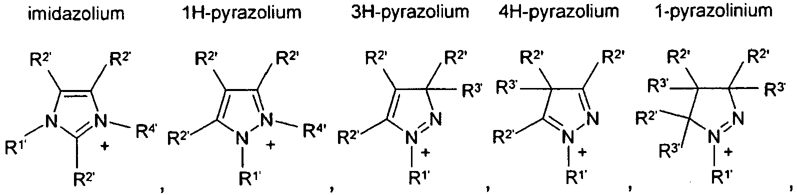 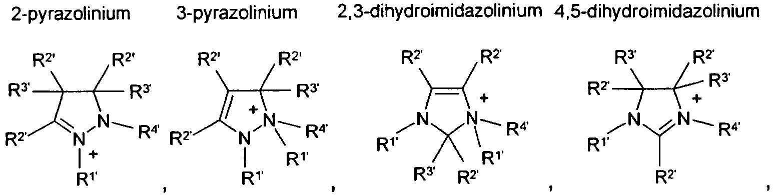 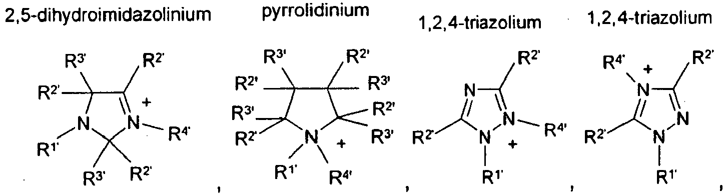 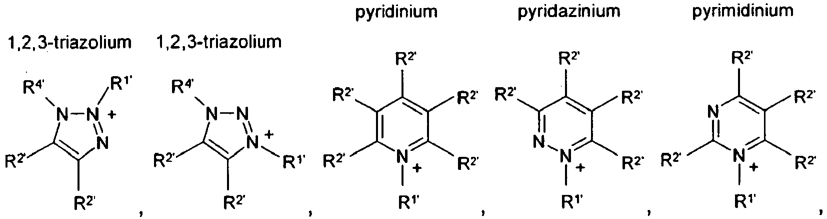 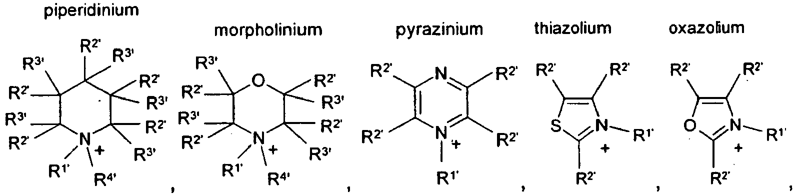 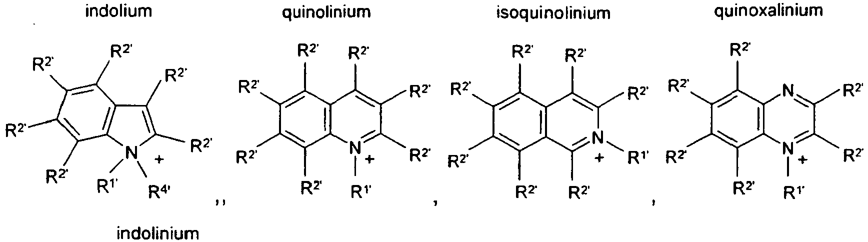 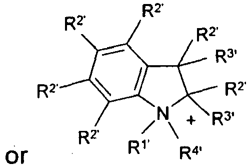 where the substituents
R¹' to R⁴' each, independently of one another, denote hydrogen, -CN, -OR', -NR'₂, -P(O)R'₂, -P(O)(OR')₂, -P(O)(NR'₂)₂, -C(O)R', -C(O)OR',
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
saturated, partially or fully unsaturated heteroaryl, heteroaryl-C₁-C₆-alkyl or aryl-C₁-C₆-alkyl,
where the substituents R^{1'}, R^{2'}, R^{3'} and/or R^{4'} together may also form a ring system,
where one or more substituents R¹' to R⁴' may be partially or fully substituted by halogens, in particular -F and/or -Cl, or -OH, -OR', -CN, -C(O)OH, -C(O)NR'₂, -SO₂NR'₂, -C(O)X, -SO₂OH, -SO₂X, -NO₂, but where R^{1'} and R^{4'} cannot simultaneously be fully substituted by halogens, and where, in the substituents R¹' to R⁴', one or two non-adjacent carbon atoms which are not bonded to the heteroatom may be replaced by atoms and/or atom groups selected from the -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

For the purposes of the present invention, fully unsaturated substituents are also taken to mean aromatic substituents.

In accordance with the invention, suitable substituents R and R² to R¹³ of the compounds of the formulae (1) to (5), besides hydrogen, are preferably: C₁- to C₂₀-, in particular C₁- to C₁₄-alkyl groups, and saturated or unsaturated, i.e. also aromatic, C₃- to C₇-cycloalkyl groups, which may be substituted by C₁- to C₆-alkyl groups, in particular phenyl.

The substituents R and R² in the compounds of the formula (1) or (2) may be identical or different here. The substituents R and R² are preferably different.

The substituents R and R² are particularly preferably methyl, ethyl, isopropyl, propyl, butyl, sec-butyl, tert-butyl, pentyl, hexyl, octyl, decyl or tetradecyl.

Up to four substituents of the guanidinium cation [C(NR⁸R⁹)(NR¹⁰R¹¹)(NR¹²R¹³)]⁺ may also be bonded in pairs in such a way that mono-, bi- or polycyclic cations are formed.

Without restricting generality, examples of such guanidinium cations are: 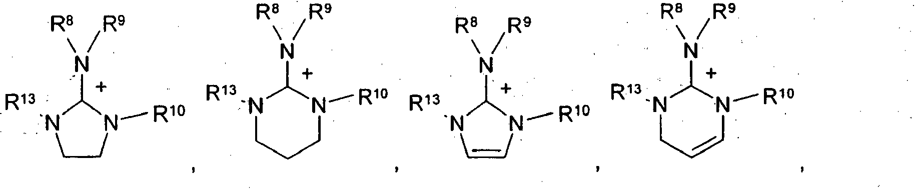 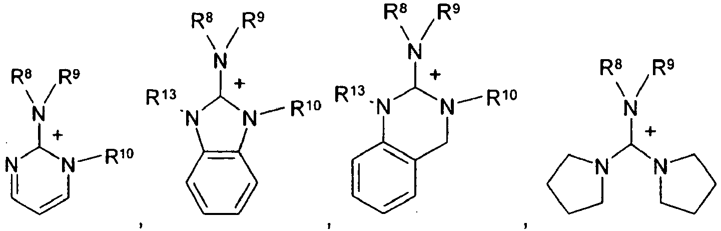 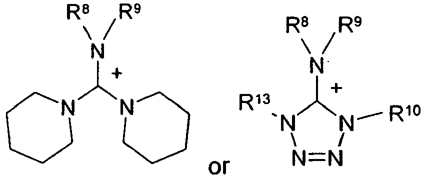 where the substituents R⁸ to R¹⁰ and R¹³ can have a meaning or particularly preferred meaning indicated above.
If desired, the carbocyclic or heterocyclic rings of the guanidinium cations indicated above may also be substituted by C₁- to C₆-alkyl, C₁- to C₆-alkenyl, NO₂, F, Cl, Br, I, OH, C₁-C₆-alkoxy, SCF₃, SO₂CF₃, COOH, SO₂NR'₂, SO₂X' or SO₃H, where X and R' have a meaning indicated above, substituted or unsubstituted phenyl or an unsubstituted or substituted heterocycle.

Up to four substituents of the uronium cation [(R³R⁴N)-C(=OR⁵)(NR⁶R⁷)]⁺ or thiouronium cation [(R³R⁴N)-C(=SR⁵)(NR⁶R⁷)]⁺ may also be bonded in pairs in such a way that mono-, bi- or polycyclic cations are formed.

Without restricting generality, examples of such cations are indicated below, where Y = O or S: 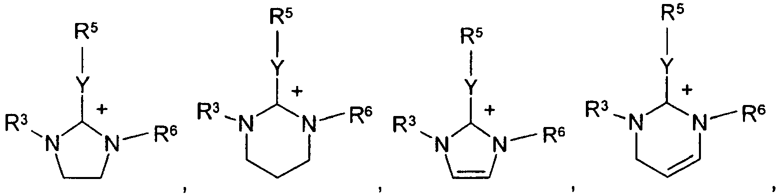 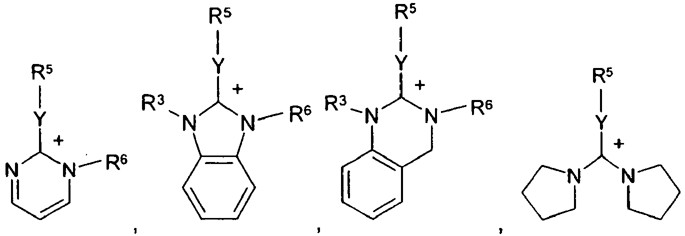 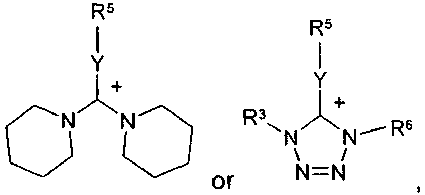 where the substituents R³, R⁵ and R⁶ can have a meaning or particularly preferred meaning indicated above.
If desired, the carbocyclic or heterocyclic rings of the cations indicated above may also be substituted by C₁- to C₆-alkyl, C₁- to C₆-alkenyl, NO₂, F, Cl, Br, I, OH, C₁-C₆-alkoxy, SCF₃, SO₂CF₃, COOH, SO₂NR'₂, SO₂X or SO₃H or substituted or unsubstituted phenyl or an unsubstituted or substituted heterocycle, where X and R' have a meaning indicated above.

The substituents R³ to R¹³ are each, independently of one another, preferably a straight-chain or branched alkyl group having 1 to 10 C atoms. The substituents R³ and R⁴, R⁶ and R⁷, R⁸ and R⁹, R¹⁰ and R¹¹ and R¹² and R¹³ in compounds of the formulae (3) to (5) may be identical or different. R³ to R¹³ are particularly preferably each, independently of one another, methyl, ethyl, n-propyl, isopropyl, n-butyl, tert-butyl, sec-butyl, phenyl or cyclohexyl, very particularly preferably methyl, ethyl, n-propyl, isopropyl or n-butyl.

In accordance with the invention, suitable substituents R^{1'} to R^{4'} of com-pounds of the formula (6), besides hydrogen, are preferably: C₁- to C₂₀, in particular C₁- to C₁₂-alkyl groups, and saturated or unsaturated, i.e. also aromatic, C₃- to C₇-cycloalkyl groups, which may be substituted by C₁- to C₆-alkyl groups, in particular phenyl.

The substituents R^{1'} and R^{4'} are each, independently of one another, particularly preferably methyl, ethyl, isopropyl, propyl, butyl, sec-butyl, tert-butyl, pentyl, hexyl, octyl, decyl, cyclohexyl, phenyl or benzyl. They are very particularly preferably methyl, ethyl, n-butyl or hexyl. In pyrrolidinium, piperidinium or indolinium compounds, the two substituents R^{1'} and R^{4'} are preferably different

The substituent R^{2'} or R^{3'} is in each case, independently of one another, in particular hydrogen, methyl, ethyl, isopropyl, propyl, butyl, sec-butyl, tert-butyl, cyclohexyl, phenyl or benzyl. R^{2'} is particularly preferably hydrogen, methyl, ethyl, isopropyl, propyl, butyl or sec-butyl. R^{2'} and R^{3'} are very particularly preferably hydrogen.
The C₁-C₁₂-alkyl group is, for example, methyl, ethyl, isopropyl, propyl, butyl, sec-butyl or tert-butyl, furthermore also pentyl, 1-, 2- or 3-methylbutyl, 1,1-, 1,2- or 2,2-dimethylpropyl, 1-ethylpropyl, hexyl, heptyl, octyl, nonyl, decyl, undecyl or dodecyl. Optionally difluoromethyl, trifluoromethyl, pentafluoroethyl, heptafluoropropyl or nonafluorobutyl.

A straight-chain or branched alkenyl having 2 to 20 C atoms, in which a plurality of double bonds may also be present, is, for example, allyl, 2- or 3-butenyl, isobutenyl, sec-butenyl, furthermore 4-pentenyl, isopentenyl, hexenyl, heptenyl, octenyl, -C₉H₁₇, -C₁₀H₁₉ to -C₂₀H₃₉; preferably allyl, 2- or 3-butenyl, isobutenyl, sec-butenyl, furthermore preferably 4-pentenyl, isopentenyl or hexenyl.

A straight-chain or branched alkynyl having 2 to 20 C atoms, in which a plurality of triple bonds may also be present, is, for example, ethynyl, 1- or 2-propynyl, 2- or 3-butynyl, furthermore 4-pentynyl, 3-pentynyl, hexynyl, heptynyl, octynyl, -C₉H₁₅, -C₁₀H₁₇ to -C₂₀H₃₇, preferably ethynyl, 1- or 2-propynyl, 2- or 3-butynyl, 4-pentynyl, 3-pentynyl or hexynyl. Aryl-C₁-C₆-alkyl denotes, for example, benzyl, phenylethyl, phenylpropyl, phenylbutyl, phenylpentyl or phenylhexyl, where both the phenyl ring and also the alkylene chain may be partially or fully substituted, as described above, by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂.

Unsubstituted saturated or partially or fully unsaturated cycloalkyl groups having 3-7 C atoms are therefore cyclopropyl, cyclobutyl, cyclopentyl, cyclohexyl, cycloheptyl, cyclopentenyl, cyclopenta-1,3-dienyl, cyclohexenyl, cyclohexa-1,3-dienyl, cyclohexa-1,4-dienyl, phenyl, cycloheptenyl, cyclohepta-1,3-dienyl, cyclohepta-1,4-dienyl or cyclohepta-1,5-dienyl, each of which may be substituted by C₁- to C₆-alkyl groups, where the cycloalkyl group or the cycloalkyl group substituted by C₁- to C₆-alkyl groups may in turn also be substituted by halogen atoms, such as F, Cl, Br or I, in particular F or Cl, or by -OH, -OR', -CN, -C(O)OH, -C(O)NR'₂, -SO₂NR'₂, -C(O)X, -SO₂OH, -SO₂X, -NO₂.

In the substituents R, R² to R¹³ or R^{1'} to R^{4'}, one or two non-adjacent carbon atoms which are not bonded in the α-position to the heteroatom may also be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = non-, partially or perfluorinated C₁- to C₆-alkyl, C₃- to C₇-cycloalkyl, unsubstituted or substituted phenyl.

Without restricting generality, examples of substituents R, R² to R¹³ and R^{1'} to R^{4'} modified in this way are:
-OCH₃, -OCH(CH₃)₂, -CH₂OCH₃, -CH₂-CH₂-O-CH₃, -C₂H₄OCH(CH₃)₂, -C₂H₄SC₂H₅, -C₂H₄SCH(CH₃)₂, -S(O)CH₃, -SO₂CH₃, -SO₂C₆H₅, -SO₂C₃H₇, -SO₂CH(CH₃)₂, -SO₂CH₂CF₃, -CH₂SO₂CH₃, -O-C₄H₈-O-C₄H₉, -CF₃, -C₂F₅, -C₃F₇, -C₄F₉, -C(CF₃)₃, -CF₂SO₂CF₃, -C₂F₄N(C₂F₅)C₂F₅, -CHF₂, -CH₂CF₃, -C₂F₂H₃, -C₃FH₆, -CH₂C₃F₇, -C(CFH₂)₃, -CH₂C(O)OH, -CH₂C₆H₅, -C(O)C₆H₅ or P(O)(C₂H₅)₂.

In R', C₃- to C₇-cycloalkyl is, for example, cyclopropyl, cyclobutyl, cyclopentyl, cyclohexyl or cycloheptyl.

In R', substituted phenyl denotes phenyl which is substituted by C₁- to C₆-alkyl, C₁- to C₆-alkenyl, NO₂, F, Cl, Br, I, OH, C₁-C₆-alkoxy, SCF₃, SO₂CF₃, COOH, SO₂X', SO₂NR"₂ or SO₃H, where X' denotes F, Cl or Br and R" denotes a non-, partially or perfluorinated C₁- to C₆-alkyl or C₃- to C₇-cycloalkyl as defined for R', for example o-, m- or p-methylphenyl, o-, m- or pethylphenyl, o-, m- or p-propylphenyl, o-, m- or p-isopropylphenyl, o-, m- or p-tert-butylphenyl, o-, m- or p-nitrophenyl, o-, m- or p-hydroxyphenyl, o-, m-or p-methoxyphenyl, o-, m- or p-ethoxyphenyl, o-, m-, p-(trifluoromethyl)-phenyl, o-, m-, p-(trifluoromethoxy)phenyl, o-, m-, p-(trifluoromethylsulfonyl)phenyl, o-, m- or p-fluorophenyl, o-, m- or p-chlorophenyl, o-, m- or p-bromophenyl, o-, m- or p-iodophenyl, further preferably 2,3-, 2,4-, 2,5-, 2,6-, 3,4- or 3,5-dimethylphenyl, 2,3-, 2,4-, 2,5-, 2,6-, 3,4- or 3,5-dihydroxy-phenyl, 2,3-, 2,4-, 2,5-, 2,6-, 3,4- or 3,5-difluorophenyl, 2,3-, 2,4-, 2,5-, 2,6-, 3,4- or 3,5-dichlorophenyl, 2,3-, 2,4-, 2,5-, 2,6-, 3,4- or 3,5-dibromophenyl, 2,3-, 2,4-, 2,5-, 2,6-, 3,4- or 3,5-dimethoxyphenyl, 5-fluoro-2-methylphenyl, 3,4,5-trimethoxyphenyl or 2,4,5-trimethylphenyl.

In R^{1'} to R^{4'}, heteroaryl is taken to mean a saturated or unsaturated mono-or bicyclic heterocyclic radical having 5 to 13 ring members, in which 1, 2 or 3 N and/or 1 or 2 S or O atoms may be present and the heterocyclic radical may be mono- or polysubstituted by C₁- to C₆-alkyl, C₁- to C₆-alkenyl, NO₂, F, Cl, Br, I, OH, C₁-C₆-alkoxy, SCF₃, SO₂CF₃, COOH, SO₂X', SO₂NR"₂ or SO₃H, where X' and R" have a meaning indicated above.
The heterocyclic radical is preferably substituted or unsubstituted 2- or 3-furyl, 2- or 3-thienyl, 1-, 2- or 3-pyrrolyl, 1-, 2-, 4- or 5-imidazolyl, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, furthermore preferably 1,2,3-triazol-1-, -4- or -5-yl, 1,2,4-triazol-1-, -4- or -5-yl, 1-or 5-tetrazolyl, 1,2,3-oxadiazol-4- or -5-yl 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-1H-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 1-, 2-, 3-, 4-, 5-, 6-, 7- or 8-quinolinyl, 1-, 3-, 4-, 5-, 6-, 7- or 8-isoquinolinyl, 1-, 2-, 3-, 4- or 9-carbazolyl, 1-, 2-, 3-, 4-, 5-, 6-, 7-, 8- or 9-acridinyl, 3-, 4-, 5-, 6-, 7- or 8-cinnolinyl, 2-, 4-, 5-, 6-, 7- or 8-quinazolinyl or 1-, 2- or 3-pyrrolidinyl.

Heteroaryl-C₁-C₆-alkyl is, analogously to aryl-C₁-C₆-alkyl, taken to mean, for example, pyridinylmethyl, pyridinylethyl, pyridinylpropyl, pyridinylbutyl, pyridinylpentyl, pyridinylhexyl, where the heterocyclic radicals described above may furthermore be linked to the alkylene chain in this way.

HetN⁺ is preferably 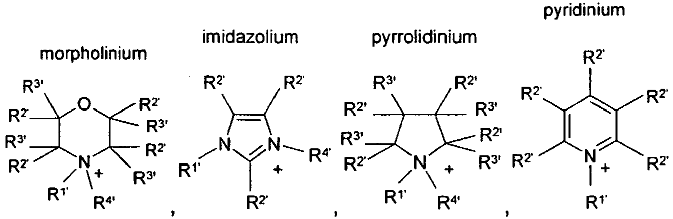 or 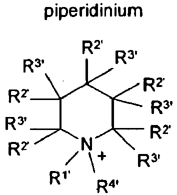 where the substituents R^{1'} to R^{4'} each, independently of one another, have a meaning described above. Morpholinium and imidazolium cations are particularly preferred in the present invention, where R¹' to R⁴' in the said cations denote, in particular, in each case independently of one another, hydrogen, straight-chain or branched alkyl having 1-20 C atoms, where one or more substituents R¹' to R⁴' may be partially substituted by -OH or -OR', where R¹' = non-, partially or perfluorinated C₁- to C₆-alkyl, C₃- to C₇-cycloalkyl, unsubstituted or substituted phenyl.

The cations of the ionic liquid according to the invention are preferably ammonium, phosphonium, imidazolium or morpholinium cations, most preferred are imidazolium cations.

Very particularly preferred substituents R, R², R¹' to R⁴' of the preferred ammonium, phosphonium, imidazolium or morpholinium cations are selected from methyl, ethyl, propyl, butyl, hexyl, decyl, dodecyl, octadecyl, ethoxyethyl, methoxyethyl, hydroxyethyl or hydroxypropyl groups.

It is preferred that the imidazolium cations are substituted by alkyl, alkenyl, aryl and/or aralkyl groups which may themselves be substituted by functional groups such as by groups containing nitrogen, sulfur and/or phosphorous wherein different oxidation states are possible. Preferred examples of these functional groups according to the invention are: amine, carboxyl, carbonyl, aldehyde, hydroxy, sulfate, sulfonate and/or phosphate groups.

One or both of the N atoms of the imidazolium ring can be substituted by identical or different substituents. Preferably both nitrogen atoms of the imidazolium ring are substituted by identical or different substituents.

It is also possible or preferred according to the invention that the imidazolium salts are additionally or exclusively substituted at one or more of the carbon atoms of the imidazolium ring.

Preferred as the substituents are C₁-C₄ alkyl groups such as methyl, ethyl, n-propyl, isopropyl, n-butyl and/or isobutyl groups. Substituents which are also preferred are C₂-C₄ alkenyl groups such as ethylene, n-propylene, isopropylene, n-butylene and/or isobutylene, also alkyl and alkenyl substituents having more than 4 C atoms are comprised wherein for example also C₅-C₁₀ alkyl or alkenyl substituents are still preferred. Due to solubility of the ionic liquid it might be favourable that these C₅-C_{10.} alkyl or alkenyl groups have one or more other substituents such as phosphate, sulfonate, amino and/or phosphate groups at their alkyl and/or alkenyl groups.

As the aryl substituents are preferred according to the invention mono-and/or bicyclic aryl groups, phenyl, biphenyl and/or naphthalene as well as derivatives of these compounds which carry hydroxy, sulfonate, sulfate, amino, aldehyde, carbonyl and/or carboxy groups. Examples of preferred aryl substituents are phenol, biphenyl, biphenol, naphthalene, naphthalene carboxylic acids, naphthalene sulfonic acids, biphenylols, biphenyl carboxylic acids, phenol, phenyl sulfonate and/or phenol sulfonic acids.

Imidazolium thiocyanates, dicyanamides, tetrafluoroborates, iodides, chlorides, bromides or hexafluorophosphates are very particularly preferably employed in the methods according to the invention, where 1-decyl-3-methylimidazolium bromide, 1-decyl-3-methylimidazolium iodide, 1-decyl-3-methylimidazolium hexafluorophosphate, 1-decyl-3-methylimidazolium tetrafluoroborate, 1-decyl-3-methylimidazolium thiocyanate, 1-decyl-3-methylimidazolium dicyanamide, 1-dodecyl-3-methylimidazolium chloride, 1-dodecyl-3-methylimidazolium bromide, 1-dodecyl-3-methylimidazolium iodide, 1-dodecyl-3-methylimidazolium hexafluorophosphate, 1-dodecyl-3-methylimidazolium tetrafluoroborate, 1-dodecyl-3-methylimidazolium thiocyanate, 1-dodecyl-3-methylimidazolium dicyanamide, 1-hexyl-3-methylimidazolium bromide, 1-hexyl-3-methylimidazolium chloride, 1-hexyl-3-methylimidazolium iodide, 1-hexyl-3-methylimidazolium hexafluorophosphate, 1-hexyl-3-methylimidazolium tetrafluoroborate, 1-hexyl-3-methylimidazolium thiocyanate, 1-hexyl-3-methylimidazolium dicyanamide, 1-octyl-3-methylimidazolium bromide, 1-octyl-3-methylimidazolium iodide, 1-octyl-3-methylimidazolium hexafluorophosphate, 1-octyl-3-methylimidazolium tetrafluoroborate, 1-octyl-3-methylimidazolium thiocyanate, 1-octyl-3-methylimidazolium dicyanamide, 1-butyl-3-methylimidazolium bromide, 1-butyl-3-methylimidazolium iodide, 1-butyl-3-methylimidazolium hexafluorophosphate, 1-butyl-3-methylimidazolium tetrafluoroborate, 1-butyl-3-methylimidazolium thiocyanate, 1-butyl-3-methylimidazolium dicyanamide, 1-ethyl-3-methylimidazolium bromide, 1-ethyl-3-methylimidazolium iodide, 1-ethyl-3-methylimidazolium hexafluorophosphate, 1-ethyl-3-methylimidazolium tetrafluoroborate, 1-ethyl-3-methylimidazolium thiocyanate, 1-ethyl-3-methylimidazolium dicyanamide, are especially preferred in the method according to the invention. Most preferred are 1-butyl-3-methylimidazolium tetrafluoroborate, 1-butyl-3-methylimidazolium thiocyanate, 1-butyl-3-methylimidazolium dicyanamide, 1-ethyl-3-methylimidazolium tetrafluoroborate, 1-ethyl-3-methylimidazolium thiocyanate, 1-ethyl-3-methylimidazolium dicyanamide, 1-hexyl-3-methylimidazolium tetrafluoroborate, 1-hexyl-3-methylimidazolium thiocyanate, 1-hexyl-3-methylimidazolium dicyanamide.

The ionic liquids used according to the invention are preferably liquids, i.e. preferably they are liquids which are ionic at room temperature (about 25° C.). However, also ionic liquids can be used which are not liquid at room temperature but which then should be present in a liquid form or should be soluble in the extraction solution at the temperature at which the method of the present invention is performed.

Another aspect of the present disclosure relates to an extraction solution for the isolation of cells from a complex matrix comprising at least:
- a divalent chloride salt optionally in combination with an ionic liquid
typically in water, and/or an aqueous buffer.

The divalent chloride salts like MgCl₂ are typically present in concentrations between 0.5 and 6 M, preferably between 0.5 and 4 M, more preferably between 1 and 2 M.

ZnCl₂ can also be used in higher concentrations due to its very high water solubility. It can be used in concentrations up to about 15 M. Preferred ZnCl₂concentrations are between 1 and 10 M. This offers the possibility to create very specific extraction conditions and to directly use the extraction solution for gradient centrifugation.

The ionic liquid - if present - is typically present in concentrations between 0.5 and 100% by weight, preferably between 1 and 60 % by weight, more preferably between 7 and 40% by weight, based on the weight of mixture. The ionic liquid can be one ionic liquid or a mixture of two or more ionic liquids.

The extraction solution of the present disclosure is an aqueous solution or a buffer solution. It typically has a pH value greater than 5 and lower than 11, preferably greater than 6 and lower than 9, more preferably between 6.5 and 7.5. The extraction solution may additionally comprise up to 20% of one or more water-miscible organic solvents like ethanol.
The extraction solution might also comprise additional component like e.g. detergents.

The buffer of the present disclosure is selected from the group of phosphate buffer, phosphate buffered saline buffer (PBS), 2-amino-2-hydroxymethyl-1, 3-propanediol (TRIS) buffer, TRIS buffered saline buffer (TBS) TRIS/EDTA (TE), ACES, MES, PIPES, HEPES and Tricine. Preferred buffers are PBS and Tricine..

Yet, another aspect of the present disclosure relates to a kit for the isolation of viruses from a complex matrix comprising:
- an extraction solution according to the present disclosure and
- at least one biopolymer degrading enzyme (see above).

According to a preferred aspect of the present disclosure the at least one biopolymer degrading enzyme is selected from the group consisting of proteases, cellulases and amylases, preferably α-amylases.

The method according to the present invention offer a very mild and effective matrix lysis system. The extraction solution effectively lyses the matrix of most of the complex samples which are e.g. typical in food analysis while the target viruses remain unaffected. In spite of the quite mild matrix lysis conditions, even the very small viruses can be isolated from the complex samples. In contrast to known isolation methods, the method according to the present invention does not comprise any additional steps in which the sample is bound to a solid phase or treated with additional extraction reagents. Especially the method according to the present invention does not involve a step in which the viral particles and/or the matrix are bound to a solid phase. The only exemption is the possibility to bind to viruses to a solid phase after extraction and isolation e.g. by centrifugation.

That means the method according to the present invention preferably does only have the following steps:
a) providing a food or clinical sample,
b) incubating said sample for a time between 10 minutes and 6 hours with an extraction solution that comprises at least a divalent chloride salt in a concentration between 0.5 and 6 m, optionally in combination with an ionic liquid
c) isolating said viruses from the mixture of step b), preferably by centrifugation, affinity binding and/or filtration,
whereby between step a) and step b) and between step b) and step c) no other steps like binding to a solid phase, treatment with additional extraction solutions or reagents are performed.

Consequently, the method of the present invention offers a simple and fast way to isolate viruses from complex samples and - combined with sensitive detection methods like real time PCR - allows for fast and sensitive detection of pathogens in food, clinical and other complex samples.

The method according to the present invention typically has a recovery rate of more than 10%. That means that typically more than 10% of the viruses present in a sample can be isolated by the method according to the present invention. Optimization of the procedure can easily lead to recovery rates of more than 20%. Recovery rates can be determined by spiking the sample with a defined amount of virus prior to performing the method according to the present invention.

One important advantage of the method according to the present invention is that the amount of the sample matrix is significantly reduced. The sample matrix of a complex sample may comprise e.g. one or more of the following constituents: peptides, polypeptides, proteins (including also enzymes), carbohydrates (complex and simple carbohydrates), lipids, fatty acids, fat, nucleic acids etc.. A complex sample matrix often interferes with analytical methods or makes it even impossible to apply molecularbiological methods to analyse the sample. It has been found that with the method according to the present invention an at least partial lysis of the sample matrix is for a time between 10 minutes and 6 hours possible and the reduction of the amount of sample matrix offers the possibility to carry out the further analysis of viral contamination.

The present invention is further illustrated by the following figures and examples, however, without being restricted thereto.

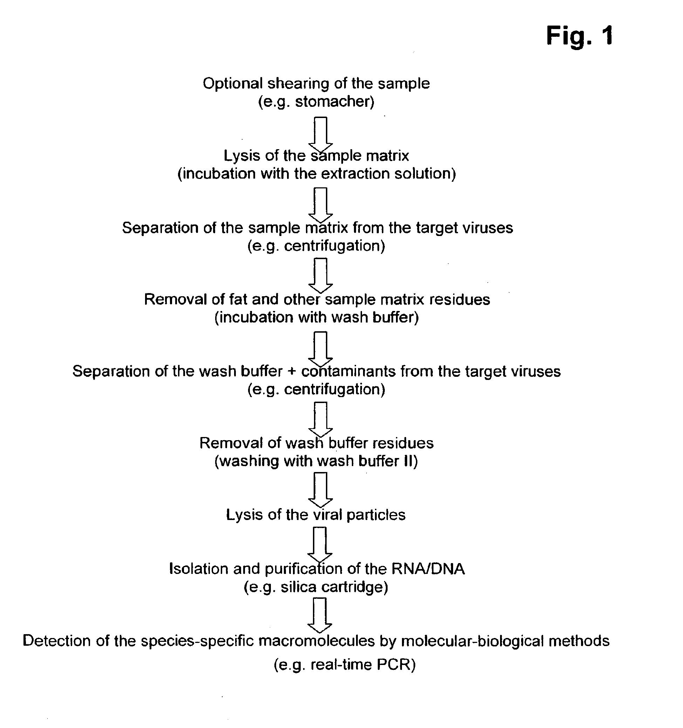

Fig. 1 gives one exemplary flow scheme for the procedural steps that can be performed when using the method according to the present invention for detecting (qualitatively and/or quantitatively) viruses in complex samples like food samples.
Fig. 2 gives an exemplary flow scheme for the procedural steps that can be performed when applying an extraction solution comprising ZnCl₂ in the method according to the present invention.

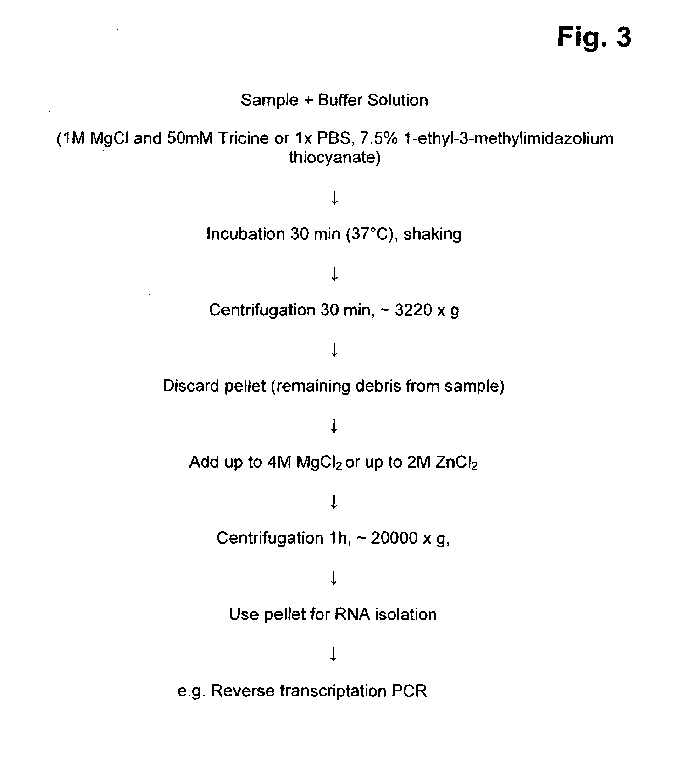

Fig. 3 gives an exemplary flow scheme for the procedural steps that can be performed when applying an extraction solution comprising an ionic liquid like 1-ethyl-3-methylimidazolium thiocyanate in the method according to the present invention.

### EXAMPLES

### Example I: Separation of bacteriophage MS2 from cheese samples.

Bacteriophage MS2 was used as a model particle for Norovirus and Rotavirus according to Dreier et al., due to the similarity of the physical and chemical properties of bacteriophage MS2 and the pathogenic Rotavirus and Norovirus. In contrast to the pathogenic viruses MS2 is easy to handle, no special safety requirements are necessary and propagation in *E. coli* does not necessitate special equipment as used in cell culture for eukaryotic cell lines, which are necessary for Norovirus and Rotavirus.

### Matrix lysis and virus isolation

Three grams of cheese (Gouda) are inoculated with 500µl MS2- phage solution (10¹⁰ PFU ml⁻¹). The lysis buffer (1M MgCl₂, 50mM Tricine) is added to a final volume of 25ml. The sample is homogenized by stomaching and incubated for 30min at 37°C and centrifuged for 20min at 4000rpm to separate remaining food debris. MgCl₂ is added to 750µl of the supernatant to a final concentration of 4M. The sample is mixed by vortexing and centrifuged at 14000rpm for 1 h. The resulting pellet is used for RNA isolation.

The PureLink® Viral RNA/ DNA Mini Kit (Invitrogen) is used according to the manufacturer's instructions. In order to enhance lysis the Proteinase K step is performed overnight. RNA bound to the silica is eluted with 20µl water. As a process control and standard for real time PCR RNA of 15µl MS2-phage solution (10¹⁰ PFU ml⁻¹) is isolated.
For cDNA synthesis 1µl of RNA is transcribed with Cloned AMV Reverse Transcriptase (Invitrogen,) according to the manufacturer's instructions. Instead of a total volume of 10µl, 20µl are produced.
The primer used for reverse transcription (MS2-TM-R) is as well as the primer used in PCR- specific for the MS2 replicase gene and described in Dreier et al., 2005.
For TaqMan® real-time PCR 5 µl of a 1:2 dilution of the cDNA is added to 20µl reaction mix containing 1 x PCR buffer (Invitrogen), 4.5mM MgCl₂, 500nM Primer (MS2-TM-F, MS2-TM-R described in Dreier et al. 2005), 250nM FAM-labelled TaqMan® probe (Dreier et al. 2005, but FAM labelled), 0.8mM NTPs (Thermo Scientific) and 0.2µl Platinum Taq DNA Polymerase (5U/ µl). The real-time PCR is preformed in the MX3000P (Stratagene) thermo-cycler as follows: Denaturation for 5min at 94°C, followed by 45 cycles of 20sec at 94°C, 30sec at 55°C, 30sec at 72°C and a final extension step at 72°C for 2min. Sample, control and negative control are performed in doublets.
For the control a serial dilution (10⁻¹- 10⁻³) is used.
For analysis of experimental data the Ct-values of the process controls and the samples are compared. The Ct-values of the sample and controls are shown in TAB.1. One log is about 3.5 Ct values therefore the recovery of MS2 equates ½ log. The difference between control and sample corresponds to a recovery of approx. 25%.

| Well Name | Assay | Threshold (dR) | Ct (dR) |
|---|---|---|---|
| 4M MgCl₂ | FAM | 1241.86 | 18.71 |
| Control | FAM | 1241.86 | 16.76 |
| Control 10⁻¹ | FAM | 1241.86 | 20.54 |
| Control 10⁻² | FAM | 1241.86 | 23.47 |
| Control 10⁻³ | FAM | 1241.86 | 27.29 |
| Neg. control | FAM | 1241.86 | No Ct |

The reduction from initially 3 gram of the applied food matrix (Gouda cheese) during the lysis step in the lysis buffer (1M MgCl₂ and 50mM Tricine) resulted in a pellet of approx. 15-25µl volume after centrifugation.

### Example II: Separation of bacteriophage MS2 from cheese samples.

### Matrix lysis and virus isolation

For inoculation of 6.5 gram sample of cheese (Gouda) 1ml MS2- phages (10¹⁰ PFU ml⁻¹) are used. Lysis buffer (1x PBS, 7.5% 1-ethyl-3-methylimidazolium thiocyanate) is added to a final volume of 45ml. The sample homogenized by stomaching and incubated for 30min at 37°C and centrifuged for 30min at 3200rpm.
An amount of 0.5 ml 4M MgCl₂ is added to 1 ml of the supernatant. The sample is mixed by vortexing and centrifuged at 14000rpm for 1 h. The resulting pellet is used for RNA isolation.
The PureLink® Viral RNA/ DNA Mini Kit (Invitrogen) is used according to the manufacturer's instructions. In order to enhance lyses the Proteinase K step is done overnight. RNA bound to the silica is eluted with 20µl water. As a process control and standard for real time PCR RNA of 22.2µl MS2-phage solution (10¹⁰ PFU ml⁻¹) is isolated.
For cDNA synthesis 1µl of RNA is transcribed with Cloned AMV Reverse Transcriptase (Invitrogen,) according to the manufacturer's instructions. The primer used for reverse transcription (MS2-TM-R) is- as well as the primer used in PCR- specific for the MS2 replicase gene and described in Dreier et al. 2005.
Five µl of the cDNA is added to 20µl reaction mix containing 1 x PCR buffer (Invitrogen), 4.5mM MgCl₂, 500nM Primer (MS2-TM-F, MS2-TM-R described in Dreier et al. 2005), 250nM FAM-labelled TaqMan® probe (Dreier et al. 2005, but FAM labelled), 0.8mM NTPs (Thermo scientific) and 0.2µl Platinum Taq DNA Polymerase (5U/ µl). The real-time PCR is preformed in the MX3000 P (Stratagene) thermo-cycler as follows: denaturation for 5min at 94°C, followed by 45 cycles of 20sec at 94°C, 30sec at 55°C, 30sec at 72°C and a final extension at 72°C for 2min. Sample, control and negative control are used in duplex.
For analysis of experimental data the Ct-values of the process controls and the samples are compared. The Ct-values of the sample and controls are shown in TAB.2. One log is about 3.5 Ct values therefore the difference between control and sample corresponds to a recovery of approx. 17%.

| Well Name | Assay | Replicate | Threshold (dR) | Ct (dR) |
|---|---|---|---|---|
| Control | FAM | 2 | 326.273 | 17.38 |
| Sample | FAM | 4 | 326.273 | 20.27 |

The reduction from initially 3 gram of the applied food matrix (Gouda cheese) during the lysis step in the lysis buffer (1x PBS, 7.5% 1-ethyl-3-methylimidazolium thiocyanate) resulted in a pellet of approx. 50-100µl volume after centrifugation.

### Example III: Separation of bacteriophage MS2 from egg samples.

A final volume of 45ml of Lysis buffer (1 M MgCl and 50mM Tricine) and a 6.5 gram sample of egg are mixed by stomaching. 500µl of the mixture are inoculated with 600µl MS2- phage solution (10¹⁰ PFU ml⁻¹) and incubated for 30min at 37°C and centrifuged for 30min at 3200rpm. Subsequently ZnCl₂ (2M final concentration) is added to he supernatant and incubated for 15min at 30°C. The sample is centrifuged at 14000rpm for 45min and the resulting pellet is used for RNA isolation.
The PureLink® Viral RNA/ DNA Mini Kit (Invitrogen) is used for RNA Isolation of the pellet and the process control according to the manufacturer's instructions. RNA bound to the silica is eluted with 20µl water. For cDNA synthesis 1µl of RNA is transcribed with Cloned AMV Reverse Transcriptase (Invitrogen,) according to the manufacturer's instructions but in total 8µl instead of 20µl cDNA was produced.
The primer used for reverse transcription (MS2-TM-R) is- as well as the primer used in PCR- specific for the MS2 replicase gene and described in Dreier et al. 2005.
For TaqMan® real-time PCR 5 µl of the cDNA is added to 20µl reaction mix containing 1x PCR buffer (Invitrogen), 4.5mM MgCl₂, 500nM Primer (MS2-TM-F, MS2-TM-R described in Dreier et al. 2005), 250nM FAM-labelled TaqMan® probe (Dreier et al. 2005, but FAM labelled), 0.8mM NTPs (Thermo scientific) and 0.2µl Platinum Taq DNA Polymerase (5U/ µl). The real-time PCR is preformed in the MX3000 P (Stratagene) thermo-cycler as follows: denaturation for 5min at 94°C, followed by 45 cycles of 20sec at 94°C, 30sec at 55°C, 30sec at 72°C and a final extension at 72°C for 2min. Sample, control and negative control are used in duplex.
For analysis of experimental data the resulting copy numbers after real-time PCR of the process control and the samples are compared. 1.94E+08 copies are found in the sample and 7.37E+08 copies are found in the process control. Therefore the recovery rate of the sample compared with the process control is 26.3%.

The reduction from initially 6.5 gram of the applied food matrix (Egg) during the lysis step in the lysis buffer (1M MgCl₂ and 50mM Tricine) resulted in a pellet of approx. 15-25µl volume after centrifugation.

## Claims

1. Method for isolating viruses from a food or clinical sample comprising the steps of:
a) providing a food or clinical sample,
b) incubating said sample for a time between 10 minutes and 6 hours with an extraction solution that comprises at least a divalent chloride salt in a concentration between 0.5 and 6 M
c) isolating said viruses from the mixture of step b).

2. Method according to claim 1, **characterized in that** the extraction solution comprises MgCl₂ in concentrations between 0.5 and 6 M.

3. Method according to one or more of claims 1 to 2, **characterized in that** in step c) the viruses are isolated from the mixture by centrifugation and/or precipitation.

4. Method according to one or more of claims 1 to 3, **characterized in that** the sample is spiked with a defined amount of control viruses prior to step b).

5. Method according to one or more of claims 1 to 4, **characterized in that** the sample is further incubated with at least one biopolymer degrading enzyme.

6. Method according to one or more of claims 1 to 5, **characterized in that** in a further step d) the viruses are analyzed by cell counting, by PCR methods, by using lectins or by methods involving antibodies or proteins selectively binding viruses or aptamers directed to surface structures of said virus particles.

7. Method according to one or more of claims 1 to 6, **characterized in that** in step c) viruses and cells surrounded by a cell wall are isolated in parallel or in subsequent isolation steps.

8. Method according to claim 7, **characterized in that** in step c) at least two subsequent centrifugation steps are performed.

9. Method according to one or more of claims 1 to 8 **characterized in that** the viruses isolated in step c) have a diameter between 10 and 100 nm.

## Patentansprüche

1. Verfahren zur Isolation von Viren aus einer Nahrungs- oder klinischen Probe, umfassend die Schritte:
a) Bereitstellen einer Nahrungs- oder klinischen Probe,
b) Inkubieren der Probe während einer Zeit zwischen 10 Minuten und 6 Stunden mit einer Extraktionslösung, die mindestens ein zweiwertiges Chloridsalz in einer Konzentration zwischen 0,5 und 6 M enthält,
c) Isolieren der Viren aus dem Gemisch von Schritt b).

2. Verfahren nach Anspruch 1, **dadurch gekennzeichnet, dass** die Extraktionslösung MgCl₂ in Konzentrationen zwischen 0,5 und 6 M enthält.

3. Verfahren nach einem oder mehreren der Ansprüche 1 bis 2, **dadurch gekennzeichnet, dass** in Schritt c) die Viren aus dem Gemisch durch Zentrifugation und/oder Ausfällung isoliert werden.

4. Verfahren nach einem oder mehreren der Ansprüche 1 bis 3, **dadurch gekennzeichnet, dass** die Probe mit einer definierten Menge an Kontrollviren vor Schritt b) aufgestockt wird.

5. Verfahren nach einem oder mehreren der Ansprüche 1 bis 4, **dadurch gekennzeichnet, dass** die Probe weiterhin mit mindestens einem Biopolymerabbauenden Enzym inkubiert wird.

6. Verfahren nach einem oder mehreren der Ansprüche 1 bis 5, **dadurch gekennzeichnet, dass** in einem weiteren Schritt d) die Viren durch Zellzählung, durch PCR-Verfahren, durch Verwendung von Lektinen oder durch Verfahren analysiert werden, die Antikörper oder Proteine, die Viren selektiv binden, oder gegen die Oberflächenstrukturen der Viruspartikel gerichtete Aptamere beinhalten.

7. Verfahren nach einem oder mehreren der Ansprüche 1 bis 6, **dadurch gekennzeichnet, dass** in Schritt c) Viren und von einer Zellwand umgebene Zellen parallel oder in aufeinander folgenden Isolationsschritten isoliert werden.

8. Verfahren nach Anspruch 7, **dadurch gekennzeichnet, dass** in Schritt c) mindestens zwei aufeinander folgende Zentrifugationsschritte durchgeführt werden.

9. Verfahren nach einem oder mehreren der Ansprüche 1 bis 8, **dadurch gekennzeichnet, dass** die in Schritt c) isolierten Viren einen Durchmesser zwischen 10 und 100 nm aufweisen.

## Revendications

1. Méthode d'isolement de virus à partir d'un échantillon d'aliment ou clinique, comprenant les étapes consistant à :
a) fournir un échantillon d'aliment ou clinique,
b) incuber ledit échantillon pendant une durée comprise entre 10 minutes et 6 heures avec une solution d'extraction qui comprend au moins un sel de chlorure divalent selon une concentration comprise entre 0,5 et 6M,
c) isoler lesdits virus à partir du mélange de l'étape b).

2. Méthode selon la revendication 1, **caractérisée en ce que** la solution d'extraction comprend du MgCl₂ selon des concentrations comprises entre 0,5 et 6M.

3. Méthode selon l'une ou plusieurs parmi les revendications 1 à 2, **caractérisée en ce que**, dans l'étape c), les virus sont isolés à partir du mélange par centrifugation et/ou précipitation.

4. Méthode selon l'une ou plusieurs parmi les revendications 1 à 3, **caractérisée en ce que** l'échantillon est dopé par une quantité définie de virus témoins préalablement à l'étape b).

5. Méthode selon l'une ou plusieurs parmi les revendications 1 à 4, **caractérisée en ce que** l'échantillon est incubé en outre avec au moins une enzyme de dégradation de biopolymères.

6. Méthode selon l'une ou plusieurs parmi les revendications 1 à 5, **caractérisée en ce que**, dans une étape supplémentaire d), les virus sont analysés par comptage cellulaire, par des méthodes de PCR, par l'utilisation de lectines ou par des méthodes impliquant des anticorps ou des protéines se fixant sélectivement à des virus ou des aptamères dirigés contre les structures de surface desdites particules virales.

7. Méthode selon l'une ou plusieurs parmi les revendications 1 à 6, **caractérisée en ce que**, dans l'étape c), les virus et les cellules entourées d'une paroi cellulaire sont isolés en parallèle ou dans des étapes d'isolement ultérieures.

8. Méthode selon la revendication 7, **caractérisée en ce que**, dans l'étape c), au moins deux étapes de centrifugation ultérieures sont réalisées.

9. Méthode selon l'une ou plusieurs parmi les revendications 1 à 8, **caractérisée en ce que** les virus isolés dans l'étape c) possèdent un diamètre compris entre 10 et 100 nm.
